# EUROPÄISCHE PATENTANMELDUNG

(11) **EP 2 044 992 A1**
(43) Veröffentlichungstag der Anmeldung: **08.04.2009**
(21) Anmeldenummer: 07019288.5
(22) Anmeldetag: 01.10.2007
(51) Int. Cl.: B01D 15/36, B01J 20/04, B01J 20/06, C07K 1/18

(54) **Verwendung von Schichtdoppelhydroxiden zur An- bzw. Abreicherung von Biomolekülen aus flüssigen oder fluiden Medien**

(71) Anmelder: SÜD-CHEMIE AG, 80333 München (DE)
(72) Erfinder: Suck, Kirstin, Dr., 81545 München (DE); Ruf, Friedrich, Dr., 84184 Ast (DE); Sohling, Ulrich, Dr., 85356 Freising (DE); Scheper, Thomas, Dr., 30559 Hannover (DE); Kasper, Cornelia, Dr., 30159 Hannover (DE); Ralla, Kathrin, 30167 Hannover (DE)
(74) Vertreter: Westendorp, Michael Oliver

(57) **Zusammenfassung**

Beschrieben wird die Verwendung eines Schichtdoppelhydroxids ausgewählt aus der Gruppe bestehend aus natürlichen und synthetischen Hydrotalciten und Verbindungen mit einer Hydrotalcit ähnlichen Struktur, wobei das Schichtdoppelhydroxid in uncalcinierter Form vorliegt, und wobei
(i) das Mol-Verhältnis von M²⁺ zu N³⁺ bei maximal 2,5:1 liegt, und/oder
(ii) das Gewichtsverhältnis von M²⁺ zu N³⁺, berechnet als das Gewichtsverhältnis der Oxide MO zu N₂O₃, bei maximal 2,2:1 liegt, und

wobei M für ein zweiwertiges Kation M²⁺ des Metalles M und N³⁺ für ein dreiwertiges Kation des Metalles N der Hydrotalcitstruktur oder Hydrotalcit ähnlichen Struktur steht;
zur An- oder Einlagerung einer Verbindung ausgewählt aus der Gruppe bestehend aus Biomolekülen, insbesondere Peptiden, Depsipeptiden, Peptidnukleinsäuren (PNAs) und Proteinen.

## Beschreibung

Die vorliegende Erfindung betrifft ein Verfahren zur An- bzw. Abreicherung von Biomolekülen, insbesondere Peptiden, Depsipeptiden, Peptidnukleinsäuren (PNAs) und Proteinen aus flüssigen Medien mittels eines Schichtdoppelhydroxids. Insbesondere betrifft die vorliegende Erfindung die Verwendung bestimmter Schichtdoppelhydroxide zur An- oder Einlagerung mindestens einer Verbindung ausgewählt aus der vorstehenden Gruppe von Biomolekülen.

Biomoleküle, wie Enzyme, Hormone oder Antikörper, gehören zur Gruppe der Proteine, von denen fast jede biologische Reaktion abhängt. Die großtechnische Herstellung dieser Biomoleküle erfolgt heutzutage mit Hilfe der Zellkulturtechnik in Mikroorganismen oder Säugetierzellen in so genannten Fermentern. Nach diesem Fermentationsprozess werden die Zielproteine in einer Reihe von Schritten von der restlichen Zellmasse aufgereinigt. Diese Aufreinigung kann viele Schritte beinhalten und somit können die Kosten dafür bis zu 80% der Gesamtkosten des Prozesses ausmachen.

Zur Aufreinigung werden meist chromatographische Methoden verwendet, um einerseits das Zielprotein im Prozessstrom anzureichern und um andererseits Fremdproteine und andere Kontaminationen, wie Endotoxine, Viren oder DNA, abzutrennen. Werden die Chromatographiematerialien mehrfach verwendet, müssen sie fortwährend auf ihre Trennleistung oder eine eventuelle mikrobielle Kontamination überprüft werden. Durch Verwendung von Einmal-Materialien können die Kosten für Validierung und Reinigung teilweise eingespart werden. Für einen erfolgreichen Einsatz muss ein solches Einmal-Chromatographiematerial eine reproduzierbare hohe Trennleistung erbringen und einen niedrigen Investitionsaufwand pro Verbrauchseinheit erfordern. Es sollte hohe Bindungskapazitäten und eine schnelle Bindungskinetik für die interessierenden Zielproteine oder die störenden Kontaminationen aufweisen.

Hydrotalcit gehört zu der Klasse der sogenannten Schichtdoppelhydroxide (layered double hydroxides, LDH), die durch die allgemeine Formel [M²⁺₁₋ₓN³⁺ₓ(OH)₂] [Aⁿ⁻]_{x/n} · y H₂O beschrieben werden. Dabei ist M²⁺ ein zweiwertiges Erdalkali- oder Übergangsmetallion wie Mg²⁺, Ni²⁺, Cu²⁺ oder Zn²⁺, N³⁺ ein dreiwertiges Hauptgruppen- oder Übergangsmetallion wie z.B. Al³⁺, Cr³⁺, Fe³⁺ oder Ga³⁺, Aⁿ⁻ ein Anion wie z. B. NO₃⁻, CO₃²⁻, Cl⁻ oder SO₄²⁻, x eine rationale Zahl zwischen 0 und 1 und y eine positive Zahl einschließlich 0. Hydrotalcit besteht in der Regel aus Magnesium- und Aluminiumionen und Carbonat als Anion. Hydrotalcit besitzt eine kationische Schichtstruktur und könnte sich daher als Ionenaustauscher für anionische Proteine eignen. Einen Überblick über die gängigen Chromatographiematerialien für Ionenaustauscher bietet: Protein purification: principles, high resolution methods, and application, 1998 2nd edition, Seiten 164-165.

Aus dem Stand der Technik ist die Verwendung von Hydrotalcit in in der aktivierten calcinierten Form als Anionaustauscher bzw. Adsorbens bekannt. Bei der Calcinierung wird Hydrotalcit in der Regel über mehrere Stunden bei 600 °C behandelt, wobei Wasser und Kohlendioxid entweichen. Die calcinierten Hydrotalcite haben basische Eigenschaften und sind sehr hitzestabil.

In der US 2005/0196850 A1 wird die Immobilisierung des enantioselektiven Enzyms Lipase aus dem Organismus Candida rugosa auf herkömmlichem Mg-Al-Hydrotalcit beschrieben.

Die Immobilisierung des Enzyms Lipase aus Candida rugosa auf bestimmten Mg-Al-Hydrotalciten ist ebenfalls in Basyaruddin et al., Catalysis Today 93-95 (2004), Seiten 405-410 dargestellt. Dabei wurden Hydrotalcite mit der allgemeinen Formel Mg/Al-NO₃⁻ und Mg/Al-Natriumdodecylsulfat (SDS) hergestellt. Die adsorbierte Menge des Enzyms war mit 70,8% auf Mg/Al-SDS höher als auf Mg/Al-NO₃⁻ (36,9%).

Die DE 102 37 518 A1 sowie die DE 102 37 517 A1 beschreiben ein Verfahren zur Behandlung von Biomolekülen, insbesondere DNA, in flüssigen oder fluiden Medien unter Verwendung eines calcinierten Schichtdoppelhydroxids als Sorptionsmittel.

Die Literaturstelle Lotsch et al., Solid State Sciences 3 (2001), Seiten 883-886, beschäftigt sich mit der Trennung von Nukleosid-Monophosphaten unter Verwendung der preferenziellen Anionenaustauschinterkalation in Schichtdoppelhydroxiden.

Die gemäß Stand der Technik als Anionenadsorptionsmittel verwendeten Schichtdoppelhydroxide sind jedoch insbesondere zur stabilen und reversiblen An- bzw. Einlagerung von Biomolekülen aus der Gruppe der Proteine, Peptide, Depsipeptide, Peptidnukleinsäuren (PNAs), insbesondere in aktiver Form, z.B. unter Erhalt von enzymatischer Aktivität bei Enzymen oder von Bindungsspezifitäten z.B. bei Antikörpern etc., oder zur An- bzw. Abreicherung von Biomolekülen aus flüssigen oder fluiden Medien nicht ausreichend geeignet. So besteht ein ständiger Bedarf an verbesserten Materialien auf diesem Gebiet.

Aufgabe der Erfindung war es daher, stabile und kostengünstige Schichtdoppelhydroxide bereitzustellen, die eine besonders effiziente An- bzw. Einlagerung von Proteinen, insbesondere zur An- bzw. Abreicherung von Proteinen aus flüssigen oder fluiden Medien, ermöglichen. Nach einer weiteren Aufgabe sollen die an- bzw. eingelagerten Biomoleküle aus der Gruppe der Proteine, Peptide, Depsipeptide, Peptidnukleinsäuren (PNAs) möglichst in nativer oder aktiver Form immobilisiert werden, um die gewünschten Eigenschaften des Biomoleküls gegenüber dem nicht immobilisierten Ausgangs-Biomolekül möglichst unverändert zu lassen. In einer bevorzugten Aufgabenstellung soll es auch möglich sein, die abgetrennten Biomoleküle zurück zu gewinnen. Dabei sollen die Nachteile des Standes der Technik vermieden und es soll eine besonders wirkungsvolle Interaktion zwischen dem Schichtdoppelhydroxid und den Proteinen je nach Art der Verwendung sichergestellt werden.

Überraschenderweise wurde im Rahmen der vorliegenden Erfindung gefunden, dass bestimmte Schichtdoppelhydroxide mit einem spezifischen Verhältnis an M²⁺ zu N³⁺ Kationen eine besonders günstige An- oder Einlagerung von Biomolekülen, ausgewählt aus der Gruppe der Proteine, Peptide, Depsipeptide, Peptidnukleinsäuren (PNAs) aufweisen und unerwartet besser zur Immobilisierung solcher Biomoleküle in "aktiver" Form geeignet sind.

Erfindungsgemäß liegt daher
(i) das Mol-Verhältnis von M²⁺ zu N³⁺ bei maximal 2,5:1, und/oder
(ii) das Gewichtsverhältnis von M²⁺ zu N³⁺, berechnet als das Gewichtsverhältnis der Oxide MO zu N₂O₃, bei maximal 2,2:1.

Dabei steht M für ein zweiwertiges Kation M²⁺ des Metalles M und N³⁺ für ein dreiwertiges Kation des Metalles N der Hydrotalcitstruktur oder Hydrotalcit ähnlichen Struktur.

So betrifft die vorliegende Erfindung nach einem ersten Aspekt die Verwendung eines Schichtdoppelhydroxids ausgewählt aus der Gruppe bestehend aus natürlichen und synthetischen Hydrotalciten und Verbindungen mit einer Hydrotalcit ähnlichen Struktur, wobei das Schichtdoppelhydroxid in uncalcinierter Form vorliegt, und wobei
(i) das Mol-Verhältnis von M²⁺ zu N³⁺ bei maximal 2,5:1 liegt, und/oder
(ii) das Gewichtsverhältnis von M²⁺ zu N³⁺, berechnet als das Gewichtsverhältnis der Oxide MO zu N₂O₃, bei maximal 2,2:1 liegt, und
wobei M für ein zweiwertiges Kation M²⁺ des Metalles M und N³⁺ für ein dreiwertiges Kation des Metalles N der Hydrotalcitstruktur oder Hydrotalcit ähnlichen Struktur steht;

zur An- oder Einlagerung einer Verbindung, ausgewählt aus der Gruppe bestehend aus Biomolekülen, insbesondere Peptiden, Depsipeptiden, Peptidnukleinsäuren (PNAs) und Proteinen.

Die erfindungsgemäß verwendeten Schichtdoppelhydroxide sind somit ausgewählt aus der Gruppe bestehend aus natürlichen und synthetischen Hydrotalciten und Verbindungen mit einer Hydrotalcit ähnlichen Struktur. Dem Fachmann ist geläufig, was unter Hydrotalciten und Verbindungen mit einer Hydrotalcit ähnlichen Struktur zu verstehen ist. Nach einem bevorzugten Aspekt kann erfindungsgemäß, solange das erfindungswesentliche Verhältnis von M²⁺ zu N³⁺ eingehalten ist, ein Schichtdoppelhydroxid, ausgewählt aus der Gruppe der natürlichen und der synthetischen Hydrotalcite und der Hydrotalcit-ähnlichen Verbindungen verwendet werden, wie sie beispielsweise in der Literaturstelle Catalysis Today, Vol. 11 (No.2) vom 2. Dezember 1991, Seiten 173 bis 301, beschrieben sind ("hydrotacite-like compounds). Solche Verbindungen weisen eine Hydrotalcit-ähnliche Struktur auf.

Zur Herstellung der Hydrotalcite bzw. der Verbindungen mit einer Hydrotalcit ähnlichen Struktur kann dabei grundsätzlich jedes dem Fachmann geläufige Verfahren eingesetzt werden, wie es beispielsweise in der vorstehenden Literaturstelle Catalysis Today (a.a.O.) auf den Seiten 173 bis 301, insbesondere 201 bis 212, in der DE 20 61 114, den US-A-5,399,329 und 5,578,286, der DE 101 19 233 oder der WO 01/12570 sowie in Handbook of Clay Science, F Bergaya, B.K.G. Theng and G. Lagaly, Developments in Clay Science, Vol. 1, Chapter 13.1, Layered Double Hydroxides, C. Forano, T. Hibino, F. Leroux, C. Taviot-Gueho, Handbook of Clay Science, 2006 beschrieben ist.

Bevorzugt liegt das Mol-Verhältnis von M²⁺ zu N³⁺ bei maximal 2,3:1, insbesondere maximal 2,1:1, und/oder das Gewichtsverhältnis von M²⁺ zu N³⁺, berechnet als das Gewichtsverhältnis der Oxide MO zu N₂O₃, bei maximal 2,0:1, insbesondere maximal 1,85:1.

Weiter wird bevorzugt, dass das Mol-Verhältnis von M²⁺ zu N³⁺ bei mindestens 0,25:1, insbesondere mindestens 0,3:1 liegt, und/oder das Gewichtsverhältnis von M²⁺ zu N³⁺, berechnet als das Gewichtsverhältnis der Oxide MO zu N₂O₃, bei mindestens 0,22:1, insbesondere mindestens 0,26:1 liegt.

Besonders bevorzugt wird dabei ein Schichtdoppelhydroxid (LDH) der allgemeinen Summenformel

[M₁₋ₓ²⁺Nₓ³⁺(OH)₂]^{x+}[Aⁿ⁻]_{x/n} · yH₂O

verwendet, wobei M²⁺ mindestens ein zweiwertiges Metallion und N³⁺ mindestens ein dreiwertiges Metallion darstellt, Aⁿ⁻ mindestens ein Anion, x eine rationale Zahl zwischen 0 und 1, n eine positive Zahl und y eine positive Zahl einschließlich 0 bedeuten.

Allgemein kann jedes für Schichtdoppelhydroxide geeignete und dem Fachmann geläufige zweiwertige Metallion oder eine Kombination aus zwei oder mehr solcher Metallionen als M²⁺ verwendet werden. Insbesondere stellt M²⁺ ein oder mehrere aus der Gruppe von Mg²⁺, Ca²⁺, Z²⁺, M²⁺, C²⁺, Ni²⁺, Fe²⁺, Sr²⁺, Ba²⁺ und/oder Cu²⁺ dar.

Allgemein kann jedes für Schichtdoppelhydroxide geeignete und dem Fachmann geläufige dreiwertige Kation oder eine Kombination aus zwei oder mehr solcher Kationen als N³⁺ verwendet werden. Insbesondere stellt N³⁺ ein oder mehrere aus der Gruppe von Al³⁺, Mn³⁺, Co³⁺, Ni³⁺, Cr³⁺, Fe³⁺, Ga³⁺, Sc³⁺, B³⁺ und/oder dreiwertige Kationen von Seltenerdenmetallen dar.

Nach einer besonders vorteilhaften erfindungsgemäßen Ausführungsform ist M²⁺ Magnesium und N³⁺ Aluminium. Für solche Materialien haben sich besonders vorteilhafte Sorptionseigenschaften für die o.g. Gruppe der Proteine und verwandter Biomoleküle gezeigt.

Es können erfindungsgemäß auch solche Doppelschichthydroxide (LDHs) verwendet werden, die auch einwertige Kationen, wie z.B. Li⁺, enthalten, die die zweiwertigen Kationen teilweise oder ganz ersetzen können. Somit sind auch Schichtdoppelhydroxide mit einwertigen Kationen von der vorliegenden Erfindung erfasst.

Nach einer besonders vorteilhaften erfindungsgemäßen Ausführungsform ist das Schichtdoppelhydroxid ein Hydrotalcit dessen allgemeine Summenformel zwischen [Mg₂Al(OH)₆] (CO₃)_{0,5} und [Mg_{0,28}Al_{0,72}(OH)₂](CO₃)_{0,72} liegt

Es wurde nach einer vorteilhaften Ausführungsform auch gefunden, dass Materialien, die neben einer Hydrotalcitphase auch eine Boehmitphase aufweisen, besonders gut geeignet sind. Eine solche Boehmitphase tritt bei hohen Aluminiumgehalten der Hydrotalcite häufig auf. Es kann aber erfindungsgemäß auch eine Mischung eines Hydrotalcits und eines Boehmits hergestellt und verwendet werden. Somit können sowohl Mischphasen als auch Mischungen aus Materialien mit Hydrotalcitphase und Materialien mit Boehmitphase eingesetzt werden. Vorzugsweise liegt der Hydrotalcitanteil bei 55 Gew.-% oder mehr.

Die erfindungsgemäß verwendeten Schichtdoppelhydroxide liegen in nicht calcinierter Form vor. Unter Calcinierung wird dabei insbesondere eine Temperaturbehandlung verstanden, bei der die Doppelschichtstruktur ganz oder teilweise verloren geht. Nach einem bevorzugten Aspekt wird das Schichtdoppelhydroxide als uncalciniert angesehen, wenn es keine Temperaturbehandlungen bei mehr als etwa 500 °C, insbesondere mehr als 450°C, insbesondere mehr als 350°C, insbesondere mehr als 250°C, insbesondere mehr als 150°C erfahren hat.

Das Zwischenschichtanion Aⁿ⁻ ist vorzugsweise aus der Gruppe bestehend aus Carbonat, Nitrat, Halogenid, Sulfat und Phosphat oder deren Gemischen ausgewählt, wobei n eine positive ganze Zahl ist. Besonders bevorzugt ist Aⁿ⁻ (siehe Summenformel oben) Carbonat oder das Schichtdoppelhydroxid liegt in Carbonatform vor, wobei bevorzugt mindestens 50 %, bevorzugter mindestens 75 % und insbesondere bevorzugt mindestens 90 % der Zwischenschichtanionen Aⁿ⁻ Carbonationen sind.

So wurde überraschend gefunden, dass die erfindungsgemäß verwendeten uncalcinierten Doppelschichthydroxide in der Carbonatform gegenüber solchen mit anderen Zwischenschichtanionen vorteilhafte Sorptionseigenschaften bei der An- oder Einlagerung der o.g. Gruppe der Biomoleküle, insbesondere in "aktiver" bzw. nativer Form aufweisen.

Unter "aktiver" oder "nativer" Form wird dabei erfindungsgemäß verstanden, dass das Biomolekül in der an- oder eingelagerten (immobilisierten) Form an dem Schichtdoppelhydroxid möglichst die gleichen Eigenschaften und Aktivitäten aufweist, die das nicht immobilisierte (sorbierte) Biomolekül (in Lösung) aufweist. Z.B. soll möglichst bei Enzymen deren enzymatische Aktivität und Selektivität erhalten bleiben, und bei Antikörpern deren Bindungsspezifität(en).

Als weiteren Vorteil weist das hier verwendete Schichtdoppelhydroxid auf, dass es an der Luft stabil ist und somit leicht gelagert werden kann.

Nach einem bevorzugten erfindungsgemäßen Aspekt umfassen die Biomoleküle nicht Rinderserumalbumin (BSA).

Die erfindungsgemäß verwendeten Schichtdoppelhydroxide können Biomoleküle in Mengen binden, die für die technische Anwendung interessant sind. So zeigt sich im Vergleich mit einem kommerziell erhältlichen starken Anionaustauscher-Material Macro-Prep 25 Q^{®} (Bio-Rad, München) sowohl im statischen, als auch dynamischen System das verwendete Schichtdoppelhydroxid eine höhere Bindungskapazität. Als weiterer Vorteil konnte das Biomolekül im dynamischen System wieder vom Schichtdoppelhydroxid eluiert werden. Als statische Kapazität wird die maximale Beladung im Gleichgewichtszustand eines statischen, d.h. nicht durchströmten Systems mit einer langen Kontaktzeit zwischen Biomoleküllösung und Sorbens (Schichtdoppelhyroxid) bezeichnet. Im statischen System wird das Biomolekül im fluiden Medium zusammen mit dem Schichtdoppelhyroxid in einem geeigneten Gefäß inkubiert, anschließend abzentrifugiert und im Schichtdoppelhyroxid-freien Überstand die Menge an gebundenem Biomolekül bestimmt.

Die Kapazität eines Ionentauschers in Abhängigkeit von der Flussrate bezeichnet man als dynamische Kapazität. Im dynamischen System wird der Hydrotalcit in eine Chromatographiesäule gepackt, das Protein-enthaltendee flüssige Medium durch die Säule gepumpt und die Durchbruchskurve des Biomoleküls ermittelt. Die Aufnahme von Durchbruchskurven dient dazu, die Adsorption des Zielbiomoleküls auf dem gewählten Sorbens zu charakterisieren. Hierzu werden Durchbruchskurven bei unterschiedlichen Fluidgeschwindigkeiten aufgenommen und anhand dieser die dynamische Kapazität bestimmt. Zur Bestimmung der dynamischen Bindungskapazität wird die Säule mit einer Biomoleküllösung (1 mg/ml) bei steigenden Flussraten kontinuierlich beladen. Die dynamische Kapazität ergibt sich anhand der Kalkulation des Volumens zu Beginn der Beladung bis zum Eintreten des Durchbruchs der Kurve (UV-Detektor). Dies wird nachstehend in den Versuchsbeschreibungen genauer erläutert.

Das im erfindungsgemäßen Verfahren verwendete Schichtdoppelhydroxid weist vorzugsweise eine BET-Oberfläche von mehr als 15 m²/g, weiter bevorzugt mehr als 20 m²/g, besonders bevorzugt mehr als 55 m²/g, vorzugsweise von mehr als 65 m²/g, und weiter bevorzugt auch ein Porenvolumen von mehr als 0,30 ml/g, besonders bevorzugt von mehr als 0,4 ml/g, insbesondere bevorzugt im Bereich von 0,45-0,6 ml/g (bestimmt nach der BJH-Methode; kumulatives Porenvolumen für Poren mit einem Durchmesser im Bereich von 1,7 bis 300 nm) auf.

Nach einer bevorzugten erfindungsgemäßen Ausführungsform weist das Schichtdoppelhydroxid einen mittleren Porendurchmesser von mehr als etwa 10 nm auf.

Besonders bevorzugt werden erfindungsgemäß Schichtdoppelhydroxid-Partikel mit einem Durchmesser zwischen etwa 0,5 bis 100 µm, besonders bevorzugt zwischen 1 und 80 µm, insbesondere bevorzugt zwischen 4 und 60 µm verwendet. Nach einer anderen bevorzugten Ausführungsform liegt das Schichtdoppelhydroxid in Form eines Granulates vor, insbesondere mit einer mittleren Teilchengröße von 0,08 - 2,5 mm.

Gemäß einer bevorzugten Ausführungsform wird das Schichtdoppelhydroxid vor der Aufgabe des Biomoleküls im flüssigen Medium auf einen pH-Wert von 5,0 bis 10,0, insbesondere bevorzugt von 6,0 bis 9,0 equilibriert. Dazu wird das Schichtdoppelhydroxid in einem geeigneten Puffer, z. B. HEPES, aufgeschlämmt oder eine aus dem Schichtdoppelhydroxid hergestellte Filterpackung mit einem derartigen Puffer beaufschlagt. Die Konzentration des Puffers ist dabei vorzugsweise zwischen 30 bis 100 mmol/l.

Gemäß einer weiteren Ausführungsform der erfindungsgemäßen Verwendung erfolgt der Einsatz in einem Chromatographie-Verfahren. Dabei wird das Schichtdoppelhydroxid in eine Chromatographiesäule gepackt und das Biomolekül-haltige flüssige Medium auf die Säule gegeben. Die Säule kann dann von einem Elutionsmittel durchströmt werden, wobei durch Adsorptionseffekte eine Auftrennung der Proteine in unterschiedliche Fraktionen möglich ist. Dies ist insbesondere interessant für die Abtrennung von Biomolekülen, die im Fermentationsprozess hergestellt wurden.

Die Elution der auf der Säule adsorbierten Biomoleküle kann über verschiedene Verfahren erfolgen. Über einen Salzgradienten können verschiede Biomoleküle bei unterschiedlichen Salzkonzentrationen gewonnen werden. Durch den Einsatz von Salzgradienten werden die elektrostatischen Wechselwirkungen zwischen dem Schichtdoppelhydroxid und dem Biomolekül herabgesetzt. Folglich wird das Biomolekül vom Schichtdoppelhydroxid abgelöst.

Nach einer weiteren Ausführungsform werden die Biomoleküle durch Änderung des pH-Wertes eluiert. Dabei hat das Elutionsmittel einen anderen pH-Wert als das flüssige Medium.

Unter Proteinen werden im Rahmen der vorliegenden Erfindung Moleküle verstanden, die als Bausteine Aminosäuren aufweisen. Die Aminosäuren haben geladene Reste, die mit geladenen Gruppen auf der Oberfläche des Schichtdoppelhydroxids wechselwirken können.

Wie vorstehend erwähnt, lassen sich mit Hilfe der vorliegenden Erfindung Biomoleküle in besonders hohen Konzentrationen an bzw. in die Schichtdoppelhydroxide an- bzw. einlagern. In einem weiteren Schritt können die Biomoleküle durch Elution wieder isoliert bzw. aufgereinigt werden. Dieser erfindungsgemäße Vorteil kommt natürlich insbesondere bei größeren bzw. längerkettigen Oligopeptiden und Proteinen zur Geltung.

Ein bevorzugter Aspekt der vorliegenden Erfindung betrifft daher die Verwendung der vorstehend definierten Schichtdoppelhydroxide zur Entfernung bzw. Gewinnung von Biomolekülen, insbesondere Proteinen, aus flüssigen oder fluiden Medien, die mindestens ein größeres oder langkettiges Biomolekül wie nachstehend definiert enthalten. Vorzugsweise weist das größere bzw. langkettige Biomolekül, wenn es sich um ein Oligopeptid oder Protein handelt, mindestens 5 Aminosäuren, vorzugsweise mindestens 50 Aminosäuren und besonders bevorzugt mindestens 100 Aminosäuren auf.

Nicht beschränkende Beispiele geeigneter Biomoleküle sind Enzyme und Antikörper, Komponenten zur Gentherapie etc.. Vorzugsweise erfolgt die erfindungsgemäße Aufreinigung und/oder Trennung unter Erhalt der biologischen Funktion.

Nach einem besonders vorteilhaften erfindungsgemäßen Aspekt enthält die vorstehende Zusammensetzung somit mindestens ein Biomolekül mit einem Molekulargewicht von mindestens 200 D, insbesondere 10 kD, besonders bevorzugt mindestens 40 kD. Der Größe des Biomoleküls sind dabei im Rahmen der Erfindung nach oben keine Grenzen gesetzt, so dass auch sehr große Biomoleküle mit mehr als 500 kD oder mehr als 1 MD besonders vorteilhaft an- bzw. eingelagert werden können. Nach einer erfindungsgemäßen Ausführungsform können aber auch einzelne Aminosäuren an- bzw. eingelagert werden.

Unter flüssigen oder fluiden Medien werden dabei alle Medien verstanden, die ein In-Kontakt-Bringen und somit eine An- bzw. Einlagerung der Biomoleküle in das Schichtdoppelhydroxid ermöglichen. Insbesondere handelt es sich in der Praxis hierbei um wässrige bzw. wasserhaltige Medien in Form einer Lösung, Suspension, Dispersion, kolloidalen Lösung oder Emulsion.

Typische Beispiele hierfür sind Flüssigkeiten, die aus biologischen Quellen stammen oder Proteine enthalten wie z. B. Prozessbrauchwässer, Fermentationsrückstände bzw. -medien, Biomoleküle enthaltende Medien aus der medizinischen oder biologischen Forschung.

Gegebenenfalls müssen die Proben je nach Verfahren vorbereitet werden, wie z. B. durch eine Grobfiltration oder Pufferung der Biomoleküle. Diese Verfahren sind dem Fachmann geläufig.

Nach einem weiteren Aspekt betrifft die Erfindung ein Verfahren, das die folgenden Schritte umfasst:
a.) in-Kontakt-Bringen eines flüssigen oder fluiden Mediums, enthaltend mindestens ein Biomolekül, wie in einem der vorstehenden Ansprüche definiert, mit dem Schichtdoppelhydroxid wie hierin definiert;
b.) Ermöglichen der An- bzw. Einlagerung des mindestens einen Biomoleküls an bzw. in das Schichtdoppelhydroxid;
c.) Abtrennen des an- bzw. eingelagerten Biomoleküls mit dem Schichtdoppelhydroxid;
d.) gegebenenfalls Wiedergewinnung bzw. Elution des mindestens einen Biomoleküls von dem Schichtdoppelhydroxid.

Die erfindungsgemäße Verwendung zur An- bzw. Einlagerung von Biomolekülen an bzw. in Schichtdoppelhydroxide kann sowohl zur Anreicherung (d.h. Erhöhung der Konzentration des/der gewünschten Biomoleküls) als auch Abreicherung (d.h. Verringerung der Konzentration des/der gewünschten Biomoleküls) genutzt werden.

Wenn die erfindungsgemäße Verwendung auf eine Entfernung oder Entsorgung von Biomolekülen abzielt, kann in einem weiteren Schritt das die Biomoleküle enthaltende Schichtdoppelhydroxid entsorgt werden. Die Entsorgung kann dabei beispielsweise durch thermische Behandlung des Schichtdoppelhydroxids erfolgen, um die enthaltenen Biomoleküle zu entfernen, wobei nach der thermischen Desintegration der Biomoleküle das Schichtdoppelhydroxid besonders vorteilhaft entsorgt werden kann.

So ist es nach einem ersten erfindungsgemäßen Aspekt möglich, Biomoleküle gezielt aus Medien zu entfernen bzw. aufzureinigen. Dies spielt beispielsweise bei der Abwasseraufbereitung eine große Rolle, da hier in den meisten Staaten strenge gesetzliche Vorschriften zur Entfernung von Biomolekülen aus Abwässern bestehen.

Genauso ist es in vielen Fällen erwünscht, die Konzentration an Biomolekülen in einem Medium zu erhöhen bzw. diese Biomoleküle möglichst in reiner Form zu gewinnen. Beispielsweise gehört die Gewinnung bzw. Aufreinigung gewünschter Biomoleküle zu den Standardprozeduren in der biologischen und medizinischen Forschung. Dabei kann erfindungsgemäß in einem weiteren Schritt das Biomolekül von dem Schichtdoppelhydroxid wieder desorbiert bzw. gewonnen werden, wodurch das Schichtdoppelhydroxid ggf. erneut eingesetzt werden kann.

Nach einem weiteren erfindungsgemäßen Aspekt können die Schichtdoppelhydroxidteilchen über ein z. B. geeignetes Bindemittel zu größeren Agglomeraten, Granulaten bzw. Formkörpern verbunden oder auf einen Träger aufgebracht werden. Die Form und Größe solcher übergeordneter Strukturen, die die primären Schichtdoppelhydroxidteilchen enthalten, richtet sich nach der jeweils gewünschten Anwendung. Es können somit alle dem Fachmann geläufigen und im Einzelfall geeigneten Formen und Größen eingesetzt werden. Beispielsweise können in vielen Fällen Agglomerate mit einem Durchmesser von mehr als 10 µm, insbesondere mehr als 50 µm bevorzugt sein. Bei einer Schüttung für Chromatographiesäulen und dergleichen kann dabei eine Kugelform der Agglomerate vorteilhaft sein. Ein möglicher Träger ist beispielsweise Calciumcarbonat.

Auch kann jedes dem Fachmann geläufige Bindemittel verwendet werden, solange die An- bzw. Einlagerung der Proteine in bzw. an die Schichtdoppelhydroxidteilchen nicht zu stark beeinträchtigt wird bzw. die für die jeweilige Anwendung zu erfordernde Stabilität der Teilchenagglomerate bzw. Formkörper gewährleistet ist. Beispielsweise, ohne hierauf beschränkt zu sein, können als Bindemittel verwendet werden: Agar-Agar, Alginate, Chitosane, Pektine, Gelatinen, Lupinenproteinisolate oder Gluten.

Ein weiterer Aspekt der vorliegenden Erfindung betrifft eine Zusammensetzung mit einem Schichtdoppelhydroxid wie vorstehend definiert und mindestens einem Biomolekül wie vorstehend definiert.

Die Erfindung wird nun anhand der folgenden Beispiele sowie unter Bezugnahme auf die beigefügten Figuren näher erläutert. Dabei zeigt:
- Fig. 1:: Darstellung der pH-abhängigen Adsorption des Proteins Ovalbumin an ein erfindungsgemäßes Schichtdoppelhydroxid (Hydrotalcit 1) im Vergleich zum kommerziell erhältlichen Aniontauscher (Macro-Prep 25 Q^{®}; Bio-Rad, München);
- Fig. 2:: Adsorptionsisotherme von Ovalbumin an Hydrotalcit 1 bei pH 8, aufgenommen in 50 mM Tris-Puffer;
- Fig. 3:: eine Graphik, in welcher die Beladung des im erfindungsgemäßen Verfahren verwendeten Hydrotalcits 1 in Abhängigkeit von der Auftragungsgeschwindigkeit für das Modellprotein BSA (A) im Vergleich zu einem von der Firma Bio-Rad kommerziell erhältlichem Chromatographiematerial (B) dargestellt ist.

### Analysenmethoden:

### Oberfläche/Porenvolumen:

Die vorliegend angegebenen BET-Oberflächen wurden nach DIN 66131 bestimmt.

Die angegebenen (mittleren) Porenvolumina und -flächen wurden unter Verwendung eines vollautomatischen Stickstoff-Adsorptionsmessgerätes (ASAP 2000, Firma Micrometrics) nach dem Standardprogramm des Herstellers (BET, BJH (I. P. Barrett, L. G. Joyner, P. P. Haienda, J. Am. Chem. Soc. 73 (1951), 373), t-plot und DFT) bestimmt. Die prozentualen Angaben zum Anteil bestimmter Porengrößen beziehen sich auf das Gesamtporenvolumen an Poren zwischen 1,7 und 300 nm Durchmesser (BJH Adsorption Pore Distribution Report).

### Bestimmung der Teilchengrößenverteilung (nach Malvern)

Dabei handelt es sich um ein gängiges Verfahren. Es wurde ein Mastersizer der Firma Malvern Instruments Ltd, UK, entsprechend der Angaben des Herstellers eingesetzt. Die Messungen wurden mit der vorgesehenen Probenkammer ("dry powder feeder") in Luft durchgeführt und die auf das Probenvolumen bezogenen Werte (wie die durchschnittliche Teilchengröße D50) ermittelt. Der D90-Wert gibt den Wert an, bei dem 90% der Teilchen in der gemessenen Probe einen kleineren oder gleichen Teilchendurchmesser aufweisen. Entsprechend geben der D100-Wert, der D50-Wert bzw. der D10-Wert den Wert an, bei dem 100%, 50% bzw. 10% der Teilchen in der gemessenen Probe einen kleineren oder gleichen Teilchendurchmesser aufweisen.

### Wassergehalt:

Der Wassergehalt der Produkte bei 120°C wurde unter Verwendung der Methode DIN/ISO 787/2 ermittelt.

### Elementaranalyse:

Diese Analyse beruht auf dem Totalaufschluss des Schichtdoppelhydroxids bzw. des entsprechenden Produkts. Nach dem Auflösen der Feststoffe werden die Einzelkomponenten mit spezifischen Analysemethoden, wie beispielsweise ICP, analysiert und quantifiziert.

### Bestimmung der Hydrotalcit- und Boehmitphasea:

Der Anteil der kristallinen Hydrotalcit-Phase oder Boehmit-Phase kann durch quantitative Röntgenbeugung bestimmt werden. Details dieser Methode sind z.B. beschrieben im "Hand Book of Clay Science", F. Bergaya, B.K.G. Therry, G. Lagaly (Hrsg.), Elsevier, Oxford, Amsterdam, 2006, Kap. 12.1: I. Srodon, Identification and Quantitative Analysis of Clay Minerals; "X-Ray Diffraction and the Identification and Analysis of Clay Minerals", D.M. Moora und R.C. Reaynolds, Oxford University Press, New York, 1997, S. 765, hier durch Bezugnahme aufgenommen.

Die quantitative Röntgenbeugung basiert auf dem Rietveld-Verfeinerungsformalismus. Dieser Algorithmus wurde ursprünglich von H.M. Rietveld zur Verfeinerung von Kristallstrukturen entwickelt. Das Verfahren wird allgemein in der Mineralogie und z.B. der Zementindustrie für die Quantifizierung der mineralischen Phasen in unbekannten Proben verwendet. Im allgemeineren Fall lässt sich diese Methode zur quantitativen Bestimmung der Gehalte von kristallinen Verbindungen (Phasen) in Mischungen aus verschiedenen kristallinen Verbindungen (Phasen) einsetzen.

Der Rietveld-Verfeinerungsalgorithmus basiert auf einer berechneten Anpassung eines simulierten Beugungsmusters auf ein gemessenes Beugungsdiagramm. Zuerst werden die vorhandenen kristallinen Phasen (Verbindungen) durch Zuordnung von Peaks des Beugungsdiagramms bestimmt. Auf der Basis der bestimmten Mineralien wird anschließend das Beugungsdiagramm auf der Basis der Kristallstruktur der in der Probe vorhandenen Mineralien sowie der Ausrüstung und der probenspezifischen Parameter berechnet. In den nächsten Schritten werden die Parameter des Modells angepasst, um eine gute Angleichung des berechneten und des gemessenen Beugungsdiagramms zu erhalten, z.B. unter Verwendung des "least square fit"-Verfahrens. Details des Verfahrens sind z.B. beschrieben in R.A. Young: "The Rietveld Method", Oxford University Press, 1995. Das Rietveld-Verfahren kann auch für stark überlappende Reflektionen im Beugungsdiagramm angewendet werden.

Zur Anwendung dieses Verfahrens bei der Analyse von mineralischen Proben wird verwiesen z.B. auf D.K. McCarthy, "Quantitative Mineral Analysis of Clay-bearing Mixtures", in: "The Reynolds Cup" Contest. IUCr CPD Newsletter, 27, 2002, 12-16.

In der Praxis erfolgt die quantitative Bestimmung der verschiedenen kristallinen Verbindungen (Phasen) in unbekannten Proben mittels im Handel erhältlicher Software, z.B. "Seifert Auto-Quan", erhältlich von Seifert/GE Inspection Technologies, Ahrensburg, Deutschland.

### pH-Wert-Bestimmung:

Der pH-Wert von Hydrotalcit wird mit einem pH-Meter in einer wässrigen/ethanolischen Suspension bestimmt. Dazu wird eine 1:1-Mischung aus Wasser und Ethanaol (95 %) hergestellt. In einem Gefäß werden 50 g der Wasser/Ethanol-Mischung auf 0,1 g genau eingewogen. 1 g Hydrotalcit wird auf 1 mg genau eingewogen und anschließend quantitativ in das Becherglas mit der Ethanol/Wasser-Mischung überführt. Es wird dann 5 min gerührt und anschließend der pH- Wert am Instrument abgelesen.

Es wurden die nachstehenden Hydrotalcite als Ausgangsmaterial verwendet, wobei die jeweilige kommerzielle Bezugsquelle angegeben ist bzw. die Herstellung entsprechender Schichtdoppelhydroxide auch nach dem Fachmann geläufigen Verfahren (siehe oben) leicht möglich ist.

### 1. Hydrotalcit 1:

Ein Al/Mg-Hydrotalcit in der Cabonatform, erhältlich von der Firma Süd-Chemie AG unter der Bezeichnung Synthal^{®}.

### Chemische Analysen:

| | |
|---|---|
| Al₂O₃: | 20,8 Gew.-% |
| MgO: | 33,8 Gew.-% |
| Mol-Verhältnis Mg:Al: | 1,85 |
| Gew.-Verhältnis MgO:Al₂O₃ | 1,63 |

### Physikalische Analysen:

| | |
|---|---|
| pH (5% Suspension, EtOH) | 8,6 |
| Wassergehalt | 4 % |
| Spezifische Oberfläche | 61,4 m2/g |
| Partikel Durchmesser (D 50) | 6,2 µm |
| Mittlerer Porendurchmesser | 30 nm |
| Porenvolumen1 | 0,482 ml/g |

| | |
|---|---|
| ¹: kumulatives Porenvolumen nach BHJ für Poren mit einem Durchmesser zwischen 1,7 und 300 nm. | |

### 2. Hydrotalcite 2, 3 und 4:

Kommerziell erhältliche Hydrotalcite der Firma Sasol Germany GmbH, DE:

| | Hydrotalcit 2 | Hydrotalcit 3 | Hydrotalcit 4 |
|---|---|---|---|
| Bezeichnung | Pural^{®} MG 30 | Pural^{®} MG 50 | Pural^{®} MG 70 |
| Gew.-verhältnis MgO:Al₂O₃ | 0,43 | 1,0 | 2,33 |
| Mol-Verhältnis Mg zu Al | 0,49 | 1,14 | 2,66 |

### 3. Kommerziell erhältlicher Anionaustauscher (Vergleich)

Produkt Macro-Prep 25 Q^{®} der Firma Bio-Rad Laboratories GmbH, München:

| | |
|---|---|
| Macro-Prep 25 Q^{®} | Funktionelle Gruppe: -N⁺(CH₃)₃ |
| | Starkes Anionenaustausch-Material |
| | Partikelgröße: 50 µm |
| | Porendurchmesser 1000 Å |

### 3. Verwendete Modellproteine:

### Ovalbumin:

Ovalbumin (Sigma-Aldrich GmbH, Taufkirchen) ist das Hauptprotein vom weißen Bestandteil des Hühnereis (60 bis 65 %). Die molekulare Masse beträgt 45 kDa und der isoeletrische Punkt ist 4,6.

### Rinderserumalbumin (BSA):

Rinderserumalbumin (Sigma-Aldrich GmbH, Taufkirchen) gehört zur Gruppe der globulären Proteine. Es sorgt vor allem im menschlichen Organismus für die Aufrechterhaltung des kolloidosmotischen Drucks. Das Molekulargewicht beträgt 66 kDa und der isoelektrische Punkt liegt bei 4,6.

### Alkalische Phosphatase:

Dieses Enzym wird aus Kälberdarmschleimhaut gewonnen und ist kommerziell von der Firma Fluka, DE erhältlich. Der isoeletrische Punkt liegt bei ca. 6 und das Molekulargewicht beträgt 140 kDa.

### Beispiel 1. Bestimmung der Protein-Bindekapazität im statischen System

### Vorbereitung des Hydrotalcits

Zunächst werden 25 mg Hydrotalcit mit 10 ml der entsprechenden Puffer bei pH 5-9 equilibriert (siehe Tabelle I). Das im Puffer suspendierte Adsorbermaterial wird 30 min im Ultraschallbad behandelt und bei 15 Upm für eine Stunde in einem Überkopf-Mischer geschüttelt. Das Material wird anschließend bei 4000 g für 10 min abzentrifugiert und der Überstand verworfen. Zum Waschen werden 10 mL bidest. Wasser zugegeben, das Adsorbermaterial resuspendiert und anschließend 5 min bei 15 Upm geschüttelt. Der Überstand wurde nach erneutem Zentrifugieren bei 4000 g/10 min verworfen.

### Vorbereitung der Modellproteine

Von den Modellproteinen werden jeweils Stammlösungen mit einer Konzentration von 2 mg/ml in bidestilliertem Wasser angesetzt. Die Proteinlösungen werden im Verhältnis 1:1 mit den in Tabelle 1 angegebenen 100 mM Puffern der pH-Werte 5-9 zu einem Gesamtvolumen von 10 ml verdünnt, so dass die Proteinendkonzentration 1 mg/ml und die Pufferendkonzentration 50 mM ist.

**Tabelle 1: Auflistung der Puffer (Sigma-Aldrich GmbH, Taufkirchen), welche zur Equilibrierung (100 mM) und Adsorption (50 mM) verwendet wurden**

| **pH-Wert** | **Puffer** |
|---|---|
| 5 | Na-Acetat |
| 6 | BISTRIS |
| 7 | HEPES |
| 8 | Tris |
| 9 | Tris |

### Bestimmung der Proteinkonzentration über UV-Messung

Die photometrische Quantifizierung von Proteinen basiert auf der Messung der UV-Absorption der aromatischen Aminosäuren Tyrosin (Phenolgruppe, 275 nm), Tryptophan (Indolgruppe, 279 nm) und in geringerem Maße Phenylalanin (257 nm) bei 280 nm. Es können je nach der Aminosäurezusammensetzung Konzentrationen zwischen 20 und 3000 µg/ml Protein nachgewiesen werden.

Um eine unbekannte Proteinkonzentration zu ermitteln, wird zunächst die Absorption einer Kalibrationsreihe von Lösungen bekannter Proteinkonzentration bei 280 nm bestimmt. Aus dem linearen Zusammenhang zwischen Absorption und Proteinkonzentration lässt sich die unbekannte Proteinkonzentration errechnen

Für die photometrische quantitative Auswertung werden von den Modellproteinen Standardreihen in den in Tabelle 1 angegebenen 50 mM Puffern der entsprechenden pH-Werte hergestellt.

### Untersuchung der pE-Abhängigkeit der Proteinadsorption

Zu jeweils 25 mg des equilibrierten Doppelschichthydroxids bzw. Macro-Prep 25 Q^{®} (siehe oben) werden 10 ml der Proteinlösung (1 mg/ml) gegeben. Die Proben werden drei Stunden bei 0,5 Upm geschüttelt. Anschließend wird das Adsorbermaterial bei 4000 g für 10 min abzentrifugiert und der Proteingehalt im Überstand anhand der UV-Absorption bei 280 mn bestimmt. Alle Versuche wurden in Dreifachbestimmung durchgeführt.

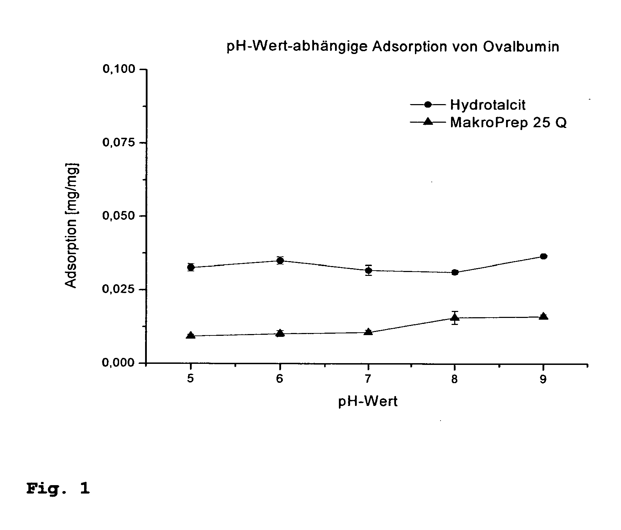

In Figur 1 sind die Ergebnisse der pH-abhängigen Adsorption des Proteins Ovalbumin an den Hydrotalcit im Vergleich zu Macro-Prep 25 Q® der Firma Bio-Rad dargestellt. Der Hydrotalcit adsorbiert ca. dreimal mehr Ovalbumin als Macro-Prep 25 Q^{®}.

### Aufnahme einer Adsorptionsisotherme

Die Aufnahme einer Adsorptionsisotherme wird in Abhängigkeit der Gleichgewichtsbeladung des Hydrotalcits von der Proteinkonzentration im Überstand untersucht. Der Versuch wurde bei einem pH-Wert von 8 in einem 50 mM Tris-Puffer durchgeführt.

Zunächst wurde Hydrotalcit wie oben beschrieben mit dem Tris-Puffer bei pH 8 equilibriert. Es wird eine Proteinstammlösung mit 10 mg/mL in 50 mM Tris-Puffer hergestellt und nach der in Tabelle 2 angegebenen Vorschrift mit dem jeweiligen Puffer verdünnt und anschließend zu 20 mg des vorbereiteten Sorbens (Doppelschichthydroxid) gegeben.

**Tabelle 2: Vorschrift zur Verdünnung der Proteinstammlösung für die Aufnahme der Protein-Adsorptionsisotherme**

| | **Proteinstammlösung [mL]** | **Puffer [mL]** | **Protein [mg]** | **C_{Ovalbumin} [mg/mL]** | **Massenverhältnis Protein/Adsorbens** |
|---|---|---|---|---|---|
| 1 | 0,79 | 14,21 | 7,95 | 0,53 | 0,318 |
| 2 | 1,59 | 13,41 | 15,9 | 1,06 | 0,636 |
| 3 | 2,40 | 12,60 | 24 | 1,60 | 0,960 |
| 4 | 6,00 | 9,00 | 60 | 4,00 | 2,400 |
| 5 | 7,95 | 7,05 | 79,5 | 5,30 | 3,180 |
| 6 | 9,90 | 5,10 | 99 | 6,60 | 3,960 |
| 7 | 12,0 | 3,00 | 120 | 8,00 | 4,800 |

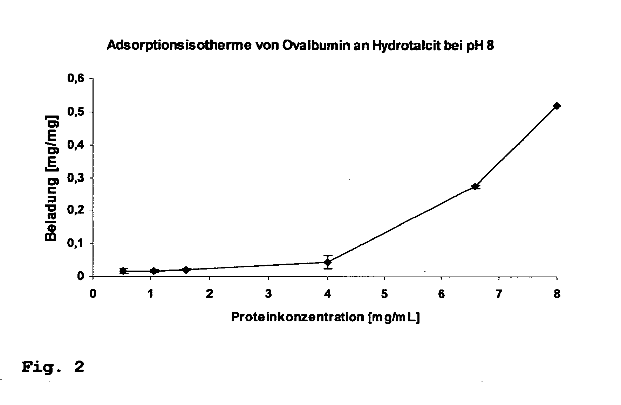

Das Protein wird für drei Stunden bei 0,5 Upm in einem Überkopf-Mischer mit dem Adsorbens inkubiert, dann bei 4000 g für 10 min abzentrifugiert und der Proteingehalt im Überstand bei 280 nm bestimmt. Die Messwerte sind als Mittelwerte von einer Dreifachbestimmung in Figur 2 dargestellt.

### Beispiel 2: Adsorption von Proteinen im dynamischen System

### Equilibrieren des Adsorbermaterials

100 mg des entsprechenden Adsorbermaterials (Hydrotalcit im Vergleich zu Macro-Prep 25 Q^{®}) werden in einem 1 mL-Eppendorfgefäß mit 1 mL des entsprechenden 50 mM Puffers (siehe Tabelle 2) suspendiert und in eine mit einem Stempel nach unten verschlossene Säule 15 mm x 50 mm (Omnifit, Cambridge, England) pipettiert. Das zweite Säulenendstück wird aufgesetzt und die Säule wird mit der HPLC-Pumpe SFD SDS 9404 (Schambeck SFD GmbH, Bad Honnef) so verbunden, dass sie von der mobilen Phase von unten nach oben durchströmt wird. Die Pumpe wird auf die Flussrate eingestellt, für welche die Kapazität des Adsorbens ermittelt werden soll. Der bewegliche Stempel der Säule wird während der Equilibrierung mit 20 mL des entsprechenden Puffers langsam handfest angezogen und anschließend um eine viertel Gewindedrehung entlastet.

### Bestimmung der Proteinbindekapazitäten

Zur Untersuchung der Beladung der Adsorbentien mit BSA im dynamischen System werden 25 mL einer BSA-Lösung (1 mg/mL) in 50 mM Puffer bei variablen Flussraten durch eine mit 100 mg Adsorbens gepackte Säule gepumpt. Die Adsorption wird bei pH 7 durchgeführt. Nach der Adsorption wird mit 25 mL Puffer gewaschen (keine pH-Wert-Änderung).

Die Elution wird mit 25 mL eines 100 mM Phosphatpuffers (pH 12) durchgeführt. Der Proteingehalt im Durchlauf wird photometrisch bei 280 nm gegen eine Standardreihe von BSA bestimmt.

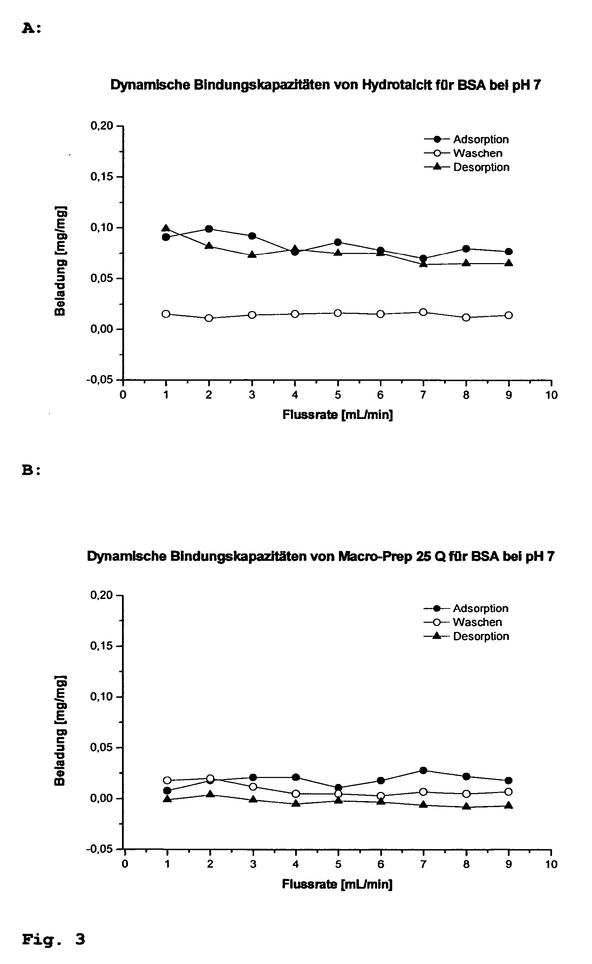

Die für den Hydrotalcit Hydrotalcit der Firma Südchemie AG und Macro-Prep 25 Q^{®} der Firma Bio-Rad ermittelten flussratenabhängigen Beladungen und Elutionen von BSA sind in Figur 3 dargestellt.

Dabei zeigte sich, dass die Bindekapazität des Hydrotalcits bis zu 10fach höher war im Vergleich zum Aniontauscher Macro-Prep 25 Q^{®}.

**Tabelle 3: Versuchsschema**

| **Equilibrieren** | **Adsorption** | **Waschen** | **Elution** |
|---|---|---|---|
| 50 mM HEPES 20 mL, pH 7 | 50 mM HEPES mit 1 mg/mL BSA, 25 mL, pH 7 | 50 mM HEPES, 25 mL, pH 7 | 100 mM Phosphat-Puffer, 25 mL, pH 12 |

### Beispiel 3: Adsorption von alkalischer Phosphatase an Doppelschichthydroxiden mit unterschiedlichem Verhältnis von M²⁺ zu N³⁺

Die Sorption von alkalischer Phosphatase an die Hydrotalcite 1 bis 4 wurde wie nachstehend angegeben durchgeführt.

### Vorbereitung der Adsorbentien

Zunächst werden 25 mg der Adsorbentien mit 10 ml der entsprechenden Puffer (50 mM) bei entsprechendem pH-Wert equilibriert (siehe Tabelle I). Das im Puffer suspendierte Adsorbermaterial wird 30 min im Ultraschallbad behandelt und bei 15 Upm für 30 min in einem Überkopf-Mischer geschüttelt. Das Material wird anschließend bei 3219 g, 10°C für 10 min abzentrifugiert und der Überstand verworfen.

Tab. 5: Auflistung der Puffer (Sigma-Aldrich GmbH, Taufkirchen), welche zur Equilibrierung und Adsorption (50 mM) verwendet wurden

| **pH-** | **Puffer** |
|---|---|
| 7 | MES |
| 9 | TRIS |

### Enzymadsorption

Zu jeweils 25 mg der equilibrierten Adsorbentien werden 10 ml der Enzymlösung (1 mg/ml) gegeben und für 30 min bei 4°C und 15 Upm im Überkopfschüttler inkubiert. Danach wird das Adsorbermaterial bei 3219 g (10°C) für 10 min abzentrifugiert und der Proteingehalt im Überstand nach der Lowry- und BCA-Methode bestimmt. Anschließend wird das Adsorbens mit 10 ml des entsprechenden Puffers für 5 min bei 4°C und 15 Upm im Überkopfschüttler gewaschen und bei 3219 g (10°C) abzentrifugiert. Vom Waschwasser wird ebenfalls der Proteingehalt ermittelt. Alle Versuche werden in Dreifachbestimmungen durchgeführt. Nachdem der Überstand restlos entfernt ist, wird das Adsorbens im Aktivitätstest eingesetzt.

Zusätzlich wurde die enzymatische Aktivität der adsorbierten alkalischen Phosphatase bestimmt. Hierzu wurde folgende Methode eingesetzt:
Aktivitätsbestimmung der geträgerten alkalischen Phosphatase (aus Kälberdarmschleimhaut )(Firma Sigma-Aldrich GmbH, Taufkirchen)

Die Aktivitätsbestimmung der alkalischen Phosphatase beruht auf der enzymatischen Spaltung von p-Nitrophenyl-Phosphat in p-Nitrophenol und anorganisches Phosphat. Durch das enstandene p-Nitrophenol tritt eine Gelbfärbung auf, welche bei 405 nm detektiert werden kann.

Um jeweils konstante Reaktionszeiten zu gewährleisten, wird die Reaktion durch Zugabe von NaOH gestoppt.

### Durchführung:

Die Aktivitätsbestimmung der geträgerten alkalischen Phosphatase wird im Falcon-Tube durchgeführt. Das geträgerte Enzym wird in 2,7 ml 1,0 M Diethanolamin-Pufferlösung (pH 9,8) suspendiert und mit 0,3 ml 150 mM p-Nitrophenyl-Phosphat-Lösung versetzt, wobei eine Gelbfärbung auftritt. Nach zwei Minuten wird die Reaktion durch Zugabe von 1 ml 1 M NaOH gestoppt. Die Suspension wird bei 3219 g (10°C) für 10 min abzentrifugiert und aus dem Überstand 34,2 µl zusammen mit 273,3 µl Pufferlösung in eine Kavität einer 96-Loch-Platte pipettiert. Die Extinktion wird bei 405 nm gemessen.

### Durchführung Standard ungeträgerte alkalische Phosphatase:

In eine Kavität einer 96-Loch-Platte werden 7,5 µl Enzymlösung und 200 µl der 1,0 M Diethanolamin-Pufferlösung (pH 9,8) pipettiert und mit 25 µl der 150 mM p-Nitrophenyl-Phosphat-Lösung versetzt. Nach zwei Minuten wird die Reaktion mit 75 µl 1 M NaOH gestoppt. Die Extinktion wird bei 405 nm gemessen.

### Durchführung Blindwert:

In eine Kavität einer 96-Loch-Platte werden 25 µl der 150 mM p-Nitrophenyl-Phosphat-Lösung, 200 µder 1,0 M Diethanolamin-Pufferlösung (pH 9,8) und 75 µl 1 M NaOH pipettiert. Die Extinktion wird bei 405 nm gemessen.

### Unit Definition:

Eine Unit setzt 1,0 µmol p-Nitrophenylphosphat pro Minute bei pH 9,8 und 37°C um.

Die Aktivität wird über das Lambert-Beersche-Gesetz aus der gemessenen Extinktion berechnet. (molarer dekadischer Extinktionskoeffiezient ε=18500 mol-1*cm-1) und im Verhältnis zur Aktivität der ungeträgerten alkalischen Phosphatase angegeben.

Die Ergebnisse sind in Tabelle 4 wiedergegeben.

**Tabelle 4:**

| | Adsorption mg Phosphatase / mg Adsorbens* | Enzymatische Aktivität | |
|---|---|---|---|
| Hydrotalcit 1 | 0,262 | ++ | |
| Hydrotalcit 2 | 0,205 | ++ | |
| Hydrotalcit 3 | 0,185 | + | |
| Hydrotalcit 4 | 0,101 | - | |

| | | | |
|---|---|---|---|
| * Mittelwert aus pH 7 und pH 9, gemessen mit BCA und Lowry Test | | | |

Es zeigt sich, dass die erfindungsgemäßen Hydrotalcite 1, 2 und 3 die beste Adsorptionsleistung zeigen. Auch ist hier die enzymatische Aktivität der alkalischen Phosphatase im Gegensatz zu dem Vergleichsmaterial 4 auch in der immobilisierten Form nachweisbar.

Die Untersuchung von zwei weiteren Hydrotalcit-Materialien, die nach herkömmlichen Synthesevorschriften mit und ohne Hydrothermalsynthese bei einem Mg:Al-Verhältnis von 1:3 hergestellt wurden, zeigten ebenfalls eine vorteilhafte Adsorption der alkalischen Phosphatase und eine gute Aktivität des immobilisierten Enzyms.

## Patentansprüche

1. Verwendung eines Schichtdoppelhydroxids ausgewählt aus der Gruppe bestehend aus natürlichen und synthetischen Hydrotalciten und Verbindungen mit einer Hydrotalcit ähnlichen Struktur, wobei das Schichtdoppelhydroxid in uncalcinierter Form vorliegt, und wobei
(i) das Mol-Verhältnis von M²⁺ zu N³⁺ bei maximal 2,5:1 liegt, und/oder
(ii) das Gewichtsverhältnis von M²⁺ zu N³⁺, berechnet als das Gewichtsverhältnis der Oxide MO zu N₂O₃, bei maximal 2,2:1 liegt, und
wobei M für ein zweiwertiges Kation M²⁺ des Metalles M und N³⁺ für ein dreiwertiges Kation des Metalles N der Hydrotalcitstruktur oder Hydrotalcit ähnlichen Struktur steht;
zur An- oder Einlagerung einer Verbindung ausgewählt aus der Gruppe bestehend aus Biomolekülen, insbesondere Peptiden, Depsipeptiden, Peptidnukleinsäuren (PNAs) und Proteinen.

2. Verwendung eines Schichtdoppelhydroxids gemäß Anspruch 1, wobei das Mol-Verhältnis von M²⁺ zu N³⁺ bei maximal 2,3:1, insbesondere maximal 2,1:1 liegt, und/oder das Gewichtsverhältnis von M²⁺ zu N³⁺, berechnet als das Gewichtsverhältnis der Oxide MO zu N₂O₃, bei maximal 2,0:1, insbesondere maximal 1,85:1 liegt.

3. Verwendung eines Schichtdoppelhydroxids gemäß Anspruch 1 oder 2, wobei das Mol-Verhältnis von M²⁺ zu N³⁺ bei mindestens 0,25:1, insbesondere mindestens 0,3:1 liegt, und/oder das Gewichtsverhältnis von M²⁺ zu N³⁺, berechnet als das Gewichtsverhältnis der Oxide MO zu N₂O₃, bei mindestens 0,22:1, insbesondere mindestens 0,26:1 liegt.

4. Verwendung eines Schichtdoppelhydroxids gemäß einem der vorstehenden Ansprüche, wobei das Schichtdoppelhydroxid neben einer Hydrotalcitphase auch eine Boehmitphase aufweist, oder Mischungen aus Materialien mit Hydrotalcitphase und Materialien mit Boehmitphase eingesetzt werden, wobei vorzugsweise der Hydrotalcitanteil bei 55 Gew.-% oder mehr liegt.

5. Verwendung eines Schichtdoppelhydroxids gemäß einem der vorstehenden Ansprüche, wobei die Proteine nicht Rinderserumalbumin (BSA) umfassen.

6. Verwendung eines Schichtdoppelhydroxids gemäß einem der vorstehenden Ansprüche, wobei
(i) M²⁺ ausgewählt ist aus der Gruppe bestehend aus Mg²⁺, Ca²⁺, Zn²⁺, Mn²⁺, Co²⁺, Ni²⁺, Fe²⁺, Sr²⁺, Ba²⁺ und Cu²⁺;
(ii) N³⁺ ausgewählt ist aus der Gruppe bestehend aus Al³⁺, Mn³⁺, Co³⁺, Ni³⁺, Cr³⁺, Fe³⁺, Ga³⁺, Sc³⁺, B³⁺ und dreiwertigen Kationen von Seltenerdenmetallen; und/oder
(iii) das Zwischenschichtanion Aⁿ⁻ ausgewählt ist aus der Gruppe bestehend aus Carbonat, Nitrat, Halogenid, Sulfat, Phosphat und Arsenat, wobei n eine positive ganze Zahl ist.

7. Verwendung eines Schichtdoppelhydroxids gemäß einem der vorstehenden Ansprüche, wobei
(i) M²⁺ das Kation Mg²⁺ ist;
(ii) N³⁺ das Kation Al³⁺ ist; und/oder
(iii) Aⁿ⁻ Carbonat ist oder das Schichtdoppelhydroxid in Carbonatform vorliegt, bevorzugt mindestens 50 %, bevorzugter mindestens 75 % und insbesondere bevorzugt mindestens 90 % der Zwischenschichtanionen Aⁿ⁻ Carbonationen sind.

8. Verwendung eines Schichtdoppelhydroxids gemäß einem der vorstehenden Ansprüche, wobei das Schichtdoppelhydroxid die Formel
[M₁₋ₓ²⁺Nₓ³⁺(OH)₂]^{x+}[Aⁿ⁻]_{x/n} · yH₂O
aufweist, worin M²⁺, N³⁺ und Aⁿ⁻ wie in den Ansprüchen 1 und 6 definiert sind, und x eine rationale Zahl zwischen 0 und 1, n eine positive Zahl und y eine positive Zahl einschließlich 0 bedeuten, und wobei
bevorzugt x von 0,28 bis 0,72 und y von 0,2 bis 10 ist,
besonders bevorzugt x 0,3 - 0,55 ist.

9. Verwendung gemäß einem der vorstehenden Ansprüche, wobei
(i) das Schichtdoppelhydroxid in Form eines Granulates vorliegt, insbesondere mit einer mittleren Teilchengröße von 0,08 - 2 mm; und/oder
(ii) der Durchmesser der einzelnen Partikel des Schichtdoppelhydroxids 0,5 bis 100 µm, bevorzugt 1 bis 80 µm und besonders bevorzugt 4 bis 60 µm beträgt; und/oder
(iii) das Schichtdoppelhydroxid eine BET-Oberfläche von mehr als 15 m²/g, bevorzugt mehr als 55 und besonders bevorzugt mehr als 65 m²/g aufweist; und/oder
(iv) das Porenvolumen nach der BJH-Methode mehr als 0,3 ml/g, bevorzugt mehr als 0,4 ml/g und besonders bevorzugt von 0,45-0,6 ml/g beträgt; und/oder
(v) der mittlere Porendurchmesser nach der BJH-Methode mehr als 10 nm beträgt; und/oder
(vi) die Partikel des Schichtdoppelhydroxids über ein Bindemittel zu größeren Agglomeraten, Granulaten oder Formkörpern verbunden oder auf einen Träger aufgebracht sind.

10. Verwendung gemäß einem der vorstehenden Ansprüche, wobei die Proteine enzymatische Aktivität aufweisen.

11. Verwendung gemäß einem der vorstehenden Ansprüche bei der Chromatographie, der Anreicherung, Abreicherung oder der Entfernung oder Gewinnung einer oder mehrerer wie im Anspruch 1 oder 10 definierter Verbindungen.

12. Verwendung gemäß einem der vorstehenden Ansprüche, wobei die wie im Anspruch 1 oder 10 definierten Verbindungen aus flüssigen oder fluiden Medien stammen, die mindestens ein wie in Anspruch 1 oder 10 definiertes Biomolekül enthalten, wobei das Biomolekül
(i) mindestens 5 Aminosäuren, vorzugsweise mindestens 50 Aminosäuren und besonders bevorzugt mindestens 100 Aminosäuren aufweist, sofern es sich um ein Oligopeptid oder Protein handelt; und/oder
(ii) ein Molekulargewicht von mindestens 200 D, bevorzugt mindestens 10 kD, weiterhin bevorzugt mindestens 40 kD, noch weiter bevorzugt mindestens 500 kD und besonders bevorzugt mindestens 1 MD aufweist.

13. Verwendung gemäß einem der vorstehenden Ansprüche zusammen mit einem Puffer,
(i) der einen pH-Wert von 5,0 bis 10,0, insbesondere von 6,0 bis 9,0 aufweist; und/oder
(ii) der eine Konzentration von 30 bis 100 mmol/l aufweist.

14. Zusammensetzung umfassend ein wie in den vorstehenden Ansprüchen definiertes Schichtdoppelhydroxid und ein wie in einem der Ansprüche 1, 10 oder 12 definiertes Biomolekül.

15. Verfahren umfassend die folgenden Schritte
(i) In-Kontakt-Bringen eines flüssigen oder fluiden Mediums, enthaltend mindestens ein wie in einem der Ansprüche 1, 10 oder 12 definiertes Biomolekül, mit einem wie in einem der vorstehenden Ansprüche definierten Schichtdoppelhydroxid als Sorptionsmittel;
(ii) Ermöglichen der An- oder Einlagerung des mindestens einen Biomoleküls an oder in das Schichtdoppelhydroxid; und
(iii) Abtrennen des an- oder eingelagerten Biomoleküls mit dem Schichtdoppelhydroxid.

16. Verfahren gemäß Anspruch 15, umfassend den weiteren Schritt der Wiedergewinnung und/oder Elution des mindestens einen Biomoleküls von dem Schichtdoppelhydroxid.

17. Verfahren gemäß Anspruch 15 oder 16, wobei vor dem Schritt (i)
a) das Schichtdoppelhydroxid mit einem Puffer auf pH 5,0 bis 9,0, insbesondere von pH 6,0 bis 8,0 equilibriert wird, wobei der Puffer optional eine Konzentration von 30 bis 100 mmol/l aufweist; und/oder
b) das Schichtdoppelhydroxid gegebenenfalls nach Ausführung des Schrittes (a) in eine Chromatographiesäule gepackt wird.

18. Verfahren gemäß einem der Ansprüche 15 bis 17 zur Anreicherung, Abreicherung oder der Entfernung oder Gewinnung einer oder mehrerer wie im Anspruch 1, 10 oder 12 definierter Verbindungen.
